Europäisches Patentamt

European Patent Office

Office européen des brevets

(19)

(11)  EP 1 493 806 A1

(12)  EUROPEAN PATENT APPLICATION

(43) Date of publication:
05.01.2005 Bulletin 2005/01

(51) Int Cl.[7]: C12N 1/04, C12N 1/38

(21) Application number: 03077079.6

(22) Date of filing: 02.07.2003

(84) Designated Contracting States:
AT BE BG CH CY CZ DE DK EE ES FI FR GB GR
HU IE IT LI LU MC NL PT RO SE SI SK TR
Designated Extension States:
AL LT LV MK

(71) Applicant: Chr. Hansen A/S
2970 Hoersholm (DK)

(72) Inventors:
• Kringelum, Borge Windel
  2750 Ballerup (DK)
• Sorensen, Niels Martin
  2450 Copenhagen SV (DK)
• Sorensen, Peter
  2635 Ishoj (DK)

(54)  **Use of compounds involved in biosynthesis of nucleic acids as cryoprotective agents**

(57)  A new type of cryoprotective agents which are useful for retaining the viability and metabolic activity of frozen or freeze-dried microbial cultures is disclosed. The cryoprotective agent comprises compounds involved in biosynthesis of nucleic acids. Methods for the preparation as well as the uses of such cultures are given. Such cultures are useful as starter cultures in the manufacturing of food and feed products. Starter cultures of the invention include culture of lactic acid bacteria, e.g. *Lactococcus* species as well as other species.

## Storage stability of frozen concentrated culture CH-N 14. With and without 3% IMP added.

Fig. 2

**Description**

**FIELD OF THE INVENTION:**

The present invention relates to the field of frozen and freeze-dried microbial cultures and comprises compounds involved in biosynthesis of nucleic acids as cryoprotective agents. In particular the invention relates to cultures obtained by the use of such agents which in addition to the cryoprotective activity also confers an increased metabolic activity of the culture when it is inoculated into the medium to be fermented or converted. Such frozen or freeze-dried cultures are useful in the manufacturing of numerous food and feed products.

**BACKGROUND OF THE INVENTION:**

Microorganisms are involved in the manufacture of food and feed products including most dairy products. Thus, bacterial cultures, in particular cultures of bacteria that are generally classified as lactic acid bacteria are essential in the making of all fermented milk products, cheese and butter. However, also cultures of certain non-bacterial micro-organisms, e.g. certain yeasts and fungi, are used to process food and feed products. Cultures of such micro-organisms may be referred to as starter cultures and they impart specific features to various dairy products by performing a number of functions. Starter cultures are widely used in the diary industry as well as in the wine manufacturing industry, the juice manufacturing industry, the meat processing industry as well as a number of other industries.

Cultures of microorganisms also find important uses in the biopreservation of foodstuffs (Andersen et al., 1997).

Commercial dairy starter cultures are generally composed of lactose and citric acid fermenting bacteria. Lactic acid bacteria designates a group of Gram positive, non-motile, microaerophilic or anaerobic bacteria which ferment sugar with the production of acids including lactic acid. The industrially most useful lactic acid bacteria are found among *Lactococcus* species, *Streptococcus* species, *Enterococcus* species, *Lactobacillus* species, *Leuconostoc* species and *Pediococcus* species.

Commonly used dairy starter culture strains of lactic acid bacteria are generally divided into mesophilic organisms having optimum growth temperatures at about 30°C and thermophilic organisms having optimum growth temperatures in the range of about 40 to about 45°C. Typical organisms belonging to the mesophilic group include *Lactococcus lactis* subsp. *lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis* and *Lactobacillus ca*sei subsp. *casei.* Thermophilic lactic acid bacterial species include as examples *Streptococcus thermophilus, Enterococcus faecium, Lactobacillus lactis, Lactobacillus helveticus, Lactobacillus delbrueckii* subsp. *bulgaricus* and *Lactobacillus acidophilus.*

The dairy starter cultures are also classified according to their specific species composition and preferred industrial use. A pure starter culture comprises only a single specie whereas a mixed culture comprises two or more different species. Starter cultures are often categorised according to the temperature at which they display the optimal growth or maximal enzymatic activity. Mesophilic starter cultures typically have an optimum around 30°C, whereas thermophilic cultures have an optimum around 40-45°C (Nielsen and Ullum, 1999). Commercial relevant mesophilic mixed cultures include:

"*O-culture*" comprising *Lactococcus lactis* subsp. *lactis* and *Lactococcus lactis* subsp. *cremoris.*
"*D-culture*" comprising *Lactococcus lactis* subsp. *lactis, Lactococcus lactis* subsp. *cremoris* and *Lactococcus lactis* subsp. *lactis* biovar. *diacetylactis.*
"*L-culture*" comprising *Lactococcus lactis* subsp. *lactis, Lactococcus lactis* subsp. *cremoris* and *Leuconostoc mesenteroides* subsp. *cremoris.*
"*LD-culture*" comprising *Lactococcus lactis* subsp. *lactis, Lactococcus lactis* subsp. *cremoris, Lactococcus lactis* subsp. *lactis* biovar. *diacetylactis* and *Leuconostoc mesenteroides* subsp. *cremoris.*

An *O-culture* is used to make cheese without holes (Cheddar, Cheshire, Feta). A *D-culture* is used to make butter. A *L-culture* is used to cheese with only small holes (cottage cheese) and curdled milk products with low $CO_2$-production. A *LD-culture* is used to make cheese with normal hole sizes, curdled milk products (junket) and sour butter. Commercially, LD-cultures are currently one of the most used mixed cultures.

Commercial relevant thermophilic mixed cultures include:

"*Yoghurt culture*" comprising *Streptococcus thermophilus* and *Lactobacillus delbrueckii* subsp. *bulgaricus*,and
"*Thermophilic cheese culture*" comprising *Streptococcus thermophilus* and *Lactobacillus helveticus.*

Whereas an *Yoghurt culture* is used to make yoghurt and special Italian cheeses, *Thermophilic cheese culture* is used to make Emmentaler cheese and special Italian cheeses.

In addition, species of *Propionibacterium* are frequently used as dairy starter cultures, in particular in the manufacture of cheese. Also organisms belonging to the *Brevibacterium* genus are commonly used as food starter cultures.

Another group of microbial starter cultures is fungal cultures, including yeast cultures and cultures of filamentous fungi, which are particularly used in the manufacture of certain types of cheese and beverage. Examples of currently used cultures of fungi include *Penicillium roqueforti, Penicillium candidum, Geotrichum candidum, Torula kefir, Saccharomyces kefir* and *Saccharomyces cerevisiae.*

Starter cultures are also widely used in the meat processing industry, e.g. for the manufacturing of various sausages and salamis.

Commercial starter cultures may commonly be distributed as frozen cultures. At the low temperatures at which such frozen cultures typically are maintained most metabolic activities in the cell ceases and cells can be maintained in this suspended, but viable, state for extended periods.

Concentrated frozen cultures are commercially very interesting since such cultures can be inoculated directly into the production container. By using such concentrated frozen cultures the end-user avoids the otherwise obligatory, time-consuming intermediary fermentation step during which the starter culture are amplified, and the end-user furthermore reduces the risk of contamination drastically. Such concentrated cultures may be referred to as direct vat set (DVS®)-cultures.

As an alternative to the concentrated frozen cultures concentrated freeze dried DVS® -cultures may be prepared. Such cultures have the additional advantage that they can be shipped without refrigeration.

In general, possible damaging effects of freezing and thawing on the viability of living cells has been ascribed to cell dehydration and the formation of ice crystals in the cytosol during freezing.

A number of cryopreservatives have been found to effect the concentration of the cytosol in a controlled and minimally injurious manner so that ice crystallization in the cytosol is precluded or minimized during freezing.

The article of F.J. Chavarri et al (Biotechnology letters, vol 10, 1, 11- 16 (1988), "Cryoprotective agents for frozen concentrated starters from non-bitter Streptococcus Lactis strains") describes that the storage viability of a frozen pure *Streptococcus lactis* culture could be improved by addition of 5% lactose or 5% sucrose. The lactose or sucrose worked as cryoprotective agents. *Streptococcus lactis* is a former name of *Lactococcus lactis* subsp. *lactis.* Similarly, the article of R. Cárcoba et al (Eur Food Res Technol (2000) 211, 433 - 437, "Influence of cryoprotectants on the viability and acidifying activity of frozen and freeze-dried cells of the novel starter strain Lactococcus lactis subsp. lactis CECT 5180") describes that the storage viability of a frozen pure *Lactococcus lactis* subsp. *lactis* culture could be improved by addition of different cryoprotective agents such as sugars (lactose, sucrose and trehalose), glutamic acid and gelatin.

Also the viability of freeze dried cultures may be improved by use of cryoprotective agents. For instance EP259739 describes a number of different cryoprotective agents for freeze-dried cultures.

These, as well as other pieces of prior art describes how various carbonhydrates, proteins as well as certain surface active agents have been be used as cryoprotective agents.

In general relatively large amounts of such cryoprotective agents are required in order to obtain the cryoprotective effect. While this may present an insignificant problem in a some settings, it may present a significant problem for food processing industries where even a small undesired deviation in the taste of the fermented or processed product that is caused by the cryoprotective agent can be detrimental, and the present inventors are not aware of any commercial available concentrated frozen cultures that comprise significant amounts of cryoprotective agents.

**SUMMARY OF THE INVENTION:**

Prior to the present invention, the present inventors believed that there were no significant storage stability problems in relation to commercially relevant concentrated frozen lactic acid bacteria cultures.

Although it is well known that most living cells suffers from freezing and the subsequent thawing the issue of viability has in general not been considered of significance in relation to commercially relevant concentrated frozen lactic acid bacteria cultures and consequently we are not aware of any commercial available concentrated frozen cultures that comprise significant amounts of cryoprotective agents. The explanation may very well be that some commercial starter cultures seem to very resistant to the damaging effects of freezing and thawing.

The present inventors has for instance performed a number of stability studies with commercial concentrated lactic acid bacteria cultures that already had been frozen for 2-3 month. In the case of studies made on 2-3 month old frozen cultures no significant degradation of activity of the cultures over a period of one year at temperature below -45°C was observed. Consequently, it was believed that commercial relevant cultures did not have significant storage stability problems.

However, the present inventors, surprisingly, observed that for instance frozen *LD-cultures* had a significant loss of activity within the first 1-3 weeks of frozen storage (as illustrated in example 1). After these weeks the further loss of activity was relatively insignificant in line with the prior known results described above.

The present inventors has thus identified hitherto unrecognised stability problems which relates to the freezing and the initial phase of the storage of some types of commercial relevant concentrated frozen lactic acid bacteria cultures, such as e.g. commercial available frozen *LD-cultures,* when the cultures are stored at the temperature provided by modern industrial freezers, typically around -50°C.

Once having identified this problem, the present inventors could start to try to solve the problem. As described herein they solved this by use of a new class of cryoprotective agents which surprisingly may help solving both the problem of stability and in addition also confers an increased metabolic activity of the culture.

Thus, in a first aspect, the invention pertains to a frozen or freeze-dried culture, characterized in that the culture comprises one or more cryoprotective agent(s) selected from the group consisting one or more compound(s) involved in the biosynthesis of nucleic acids or one or more derivative(s) of any such compounds.

The cryoprotective agent should preferably be added to the viable bacteria before they are frozen or freeze dried.

Accordingly, in a second aspect the invention relates to a method for making a frozen culture or a freeze dried culture comprising the following steps: adding one or more cryoprotective agent(s) selected from the group consisting one or more compound(s) involved in the biosynthesis of nucleic acids or one or more derivative(s) of any such compounds to viable *organisms;* freezing the material to get frozen material, and packing the frozen (or freeze dryed) material in a suitable way. In the case of making a freeze dried culture the method comprises a step where sublimation of water from the frozen material occurs prior to the packing step.

According to the present invention, there is also provided a method of preparing a food and a feed product which comprises the usage of a culture according to the invention. A particular important aspect is the provision of a method of a food product selected from a milk based product, a meat product, a vegetable product and a beverage.

A further advantage of the herein described new class of cryoprotective agents is that they retain the viability as well as the metabolic activity of the reconstituted cells and do not affect the taste of the fermented or processed product in any adverse way.

**DEFFINITIONS**

Prior to a discussion of the detailed embodiments of the invention is provided a definition of specific terms related to the main aspects of the invention.

As used herein the term "lactic acid bacterium" (LAB) designates a gram-positive, microaerophilic or anaerobic bacterium which ferments sugars with the production of acids including lactic acid (as the predominantly produced acid), acetic acid and propionic acid. The industrially most useful lactic acid bacteria are found among *Lactococcus* species (spp.), *Streptococcus* spp., *Lactobacillus* spp., *Leuconostoc* spp., *Pediococcus* spp., *Brevibacterium* spp , *Enterococcus* spp. *and Propionibacterium* spp. Additionally, lactic acid producing bacteria belonging to the group of the strict anaerobic bacteria, *bifidobacteria,* i.e. *Bifidobacterium* spp. which are frequently used as food starter cultures alone or in combination with lactic acid bacteria, are generally included in the group of lactic acid bacteria.

By the term a " concentrated" frozen or freeze-dried culture is referred to a composition that have a content of viable cells (colony forming units, CFUs) which is at least $10^8$ CFU per ml, e.g. at least $10^9$ CFU per ml, such as at least $10^{10}$ CFU per ml including at least $10^{11}$ CFU per ml, e. g. at least $10^{12}$ CFU per ml.

The term "packing" should be understood broadly. It denotes that the frozen or freeze-dried culture should be packed in order could to be provided to the user. It may be packed in a bottle, a tetra-pack@, a bag, etc.

The term "a cryoprotective agent" denotes a substance that is able to improve the resistance towards the damaging effects induced by freezing and the initial phase of the storage of the frozen or freeze-dried culture. In the present context it may be a single specific cryoprotective agents or it may be two or more different agents. Accordingly, the w/w percentage of the cryoprotective agent(s) within the culture material should be understood as the sum of the amount of cryoprotective agent(s). A preferred way to determine whether a substance is a cryoprotective agent that is able to improve resistance of the frozen culture to the damaging effects induced by freezing and the initial phase of the storage is to spilt a culture, as described herein, in two samples, add a specified amount of the cryoprotective agent to one of them, freezing both of them and measure the acidifying activity in th erelevant media (e.g. milk) of the cultures on the same day as freezing and periodically (e.g. up to one year) when kept under frozen storage. If the culture with cryoprotective agent has improved milk acidifying activity seen over the storage period the substance is a cryoprotective agent. A suitable milk acidifying activity assay is given in working examples herein.

Embodiments of the present invention is described below, by way of examples only.

**DETAILED DISCLOSURE OF THE INVENTION:**

As discussed previously, concentrated frozen or freeze-dried cultures are considered to be stable. However contrary to the general belief in the field the inventors surprisingly observed hitherto unrecognised stability problems which relates to the freezing and the initial phase of the storage of some types of commercial relevant concentrated frozen lactic acid bacteria cultures, such as e.g. commercial available frozen *LD-cultures.* It is contemplated that such stability problems will be widely found when commercial cultures are being tested appropriately.

In order to overcome this problem a number of possible agents were tested to see if they could overcome the problem. Among the agents tested were agent(s) selected from the group consisting one or more compound(s) involved in the biosynthesis of nucleic acids which previously were shown by the inventors to improve the stability of non-frozen, liquid starter cultures (WO0039281). Quite unexpectedly experiments show that such compounds are effective cryoprotective agents for certain concentrated lactic acid bacteria cultures. As it is shown in Example 2 below, the addition of one such compound, inosine-5'-monophosphate (IMP), significantly improves the resistance towards the damaging effects induced by freezing and the initial phase of the storage. The addition of IMP also significantly improves the stability of a freeze dried culture as illustrated in example 9 below. From the example 10 below it is clear that not only IMP but a wider range of agents selected from the group consisting of compound(s) involved in the biosynthesis of nucleic acids are effective as cryoprotective agents.

As illustrated in example 10 both nucleotides and nucleosides can be used as cryoprotective agents. Thus, in presently preferred embodiments, a cryoprotective compound which is useful to improve the resistance towards the damaging effects induced by freezing and the initial phase of the storage of a starterculture according to the invention is a compound selected from the group consisting of a compound involved in the biosynthesis of nucleic acids, including the group of purine bases, pyrimidine bases, nucleosides and nucleotides. Such compounds are exemplified by inosine-5'-monophosphate (IMP), adenosine-5'-monophosphate (AMP), guanosine-5'-monophosphate (GMP), uranosine-5'-monophosphate (UMP), cytidine-5'-monophosphate (CMP), adenine, guanine, uracil, cytosine, adenosine, guanosine, uridine, cytidine, hypoxanthine, xanthine, hypoxanthine, orotidine, thymidine, inosine and a derivative of any of such compounds.

In a further preferred embodiment the one or more cryoprotective agent(s) is/are selected from the group of nucleoside monophosphates. In particular embodiments wherein the cryoprotective agent is IMP are preferred.

Whereas cryoprotective agents of the cryoprotectant agents of the carbonhydrate or proteinaous type in general not is described to increase the metabolic activity of thawed or reconstituted cultures the cryoprotective agents of the present invention may in addition to their cryoprotective activity also confers an increased metabolic activity (booster effect) of the culture when it is inoculated into the medium to be fermented, processed or converted.

Thus one embodiment of the present invention is a froze or freeze-dried culture, wherein the cryoprotective agent is an agent or mixture of agents, which in addition to its cryoprotectivity has a booster effect.

In the present connection the expression "booster effect" is used to describe the situation that the cryoprotective agent confers an increased metabolic activity (booster effect) on to the thawed or reconstituted culture when it is inoculated into the medium to be fermented or converted. Viability and metabolic activity are not synonymous concepts. Commercial frozen or freeze-dried cultures may retain their viability, although they may have lost a significant portion of their metabolic activity such as e. g. their acid-producing (acidification) activity when kept stored even for shorter periods of time. Thus viability and booster effect has to be evaluated by different assays. Whereas viability is assessed by viability assays such as the determination of colony forming units, booster effect is assessed by quantifying the relevant metabolic activity of the thawed or reconstituted culture relative to the viability of the culture. The acidifying activity assay described below is one example of an assay quantifying the relevant metabolic activity of the thawed or reconstituted culture. The booster effect is further illustrated in Example 3.

Although the acid-producing activity is exemplified herein, this invention is intended to encompass the stabilization of any types of metabolic activities of a culture. Thus, the term "metabolic activity" refers to the oxygen removal activity of the cultures, its acid-producing activity, i. e. the production of e. g. lactic acid, acetic acid, formic acid and/ or propionic acid, or its metabolite producing activity such as the production of aroma compounds such as acetaldehyde, ($\alpha$-acetolactate, acetoin, diacetyl and 2,3-butylene glycol (butanediol)).

In a preferred embodiment of the invention the frozen culture contains comprises from about 0.1% to about 20% of the cryoprotective agent or mixture of agents measured as %w/w of the frozen material. It is, however, preferred to add the cryoprotective agent or mixture of agents at an amount which is in the range from 0.1% to 15%, such as within the range of 0.2% to 10%, e. g. within the range of 0.5% to 7%, such as within the range of 1% to 6% by weight, including within the range of 2% to 5% of the cryoprotective agent or mixture of agents measured as %w/w of the frozen material by weight. In a preferred embodiment the culture comprises approximately 3% of the cryoprotective agent or mixture of agents measured as %w/wof the frozen material by weight

The term "material" of the culture denotes the relevant substances of the culture including both the viable bacteria and cryoprotective agent. Possible packing is not included. Consequently, the weight of the material of the culture is not including the weight of possible packing.

In the case that the culture is a freeze-dried culture it is preferred to add the cryoprotective agent or mixture of agents at an amount which is in the range of 0.8% to 60% by weight, e. g. within the range of 0.8% to 55% by weight, such as within the range of 1.3% to 40% by weight, e. g. within the range of 3% to 30% by weight, such as within the range of 6% to 25% by weight, including within the range of 10% to 24% by weight. In a preferred embodiment the freeze-dried culture comprises approximately 16% of the cryoprotective agent or mixture of agents measured as %w/wof the freeze-dried material by weight

Additionally, the frozen or freeze-dried culture may contain further conventional additives including nutrients such as yeast extract, sugars, antioxidants, inert gases and vitamins etc. Also surfactants including Tween® compounds can be used as further additive to the culture according to the invention.

Futher examples of such conventional additives which in addition may be added to the culture according to the invention may be selected from proteins, protein hydrolysates and amino acids. Preferred suitable examples of these include the ones selected from the group consisting of Glutamic acid, Lysine, Na-glutamate, Na-caseinate, Malt extract, Skimmed milk powder, Whey powder, Yeast extract, Gluten, Collagen, Gelatin, Elastin, Keratin, and Albumins.

More preferably the conventional additives is a carbonhydrate. Suitable examples of these include the ones selected from the group consisting Pentoses (eg. Ribose, Xylose), Hexoses (eg. fructose, mannose, Sorbose), Disaccharides (eg. Sucrose, Trehalose, Melibiose, Lactulose), Oligosaccharides (eg. Raffinose), Oligofrutoses (eg. Actilight, Fribroloses), Polysaccharides (eg. Maltodextrins, Xanthan Gum, Pectin, Alginate, Microcrystalline cellulose, Dextran, PEG), and Sugar alcohols (Sorbitol, Manitol and Inositol).

Although the present invention relates to any concentrated frozen of freeze dried cultures, it in particular directed towards cultures of microorganisms that are involved in the manufacture of food and feed products including most dairy products. Preferred embodiments of the invention comprise bacterial cultures, in particular cultures of bacteria that are generally classified as lactic acid bacteria and which are essential in the making of all fermented milk products, cheese and butter. Cultures of such bacteria are often referred to as starter cultures and they impart specific features to various dairy products by performing a number of functions. However also cultures which comprise fungal cultures, including yeast cultures and cultures of filamentous fungi, which are particularly used in the manufacture of certain types of cheese and beverage are referred to as starter cultures. Also cultures, which are used to process other types of food or feed products are referred to as starter cultures. The cultures used in the manufacturing of silage are often referred to as starter cultures too.

In accordance with the invention, any starter culture organism which is of use in the food or feed industry including the dairy industry can be used. Thus, the starter culture can comprises one or more organisms selected from the group comprising a lactic acid bacterial (LAB) spp., a *Bifidobacterium* spp., a *Brevibacterium* spp., a *Propionibacterium* spp. or a fungal spp. such as a *Torula* spp. a *Penicillium* spp., a *Cryptococcus* spp., *Debraryomyces* spp., *Klyveromyces* spp. and a *Saccharomyces* spp.. Suitable cultures of the lactic acid bacterial (LAB) group include commonly used strains of a *Lactococcus* spp., a *Streptococcus* spp., a *Lactobacillus* spp. including the *Lactobacillus acidophilus, Enterococcus* spp., *Pediococcus* spp., a *Leuconostoc* spp. *Oenococcus* spp.. *Lactococcus* spp. and include the widely used *Lactococcus lactis,* including *Lactococcus lactis* subsp. *lactis* and *Lactococcus lactis* subsp. *cremoris* which are commonly used in the manufacture of cheeses with a closed texture, e. g. Cheddar, Feta and cottage cheese.

It will be appreciated, that the starter culture organism can be selected from a genetically modified strain of one or more of the above lactic acid bacterial strains or any other starter culture strains. As used herein the expression "genetically modified bacterium" is used in the conventional meaning of that term i. e. it refers to strains obtained by subjecting a bacterial strain to any conventionally used mutagenization treatment including treatment with a chemical mutagen such as ethanemethane sulphonate (EMS) or Nmethyl-N'-nitro-N-nitroguanidine (NTG), to UV light or to spontaneously occurring mutants, including classical mutagenesis. Futhermore it is possible to provide the genetically modified bacterium by random mutagenesis followed by selection of the spontaneously occurring mutants, i. e. without the use of recombinant DNA-technology. It is further envisaged that mutants of lactic acid bacteria and other potential useful starter culture organisms can be provided by such technology including site-directed mutagenesis and PCR techniques and other *in vitro* or *in vivo* modifications of specific DNA sequences once such sequences have been identified and isolated. Thus it is further contemplated that useful starter culture organisms can be obtained by use of recombinant DNA-technology. In particular the possibility to obtain useful starter culture organisms by recombination of DNA-sequences that are on beforehand were were inherent to the particular organism, i.e. self-cloning, is attractive from a food-regulation point of view.

As it is usual in the dairy industry, the starter culture may comprise a mixture of strains including a mixture of strains of different lactic acid bacterial species, such as e. g. a mixture of Streptococcus thermophilus and *Lactobacillus delbrueckii* subsp. *bulgaricus.*

Commonly used dairy starter culture strains generally divided into "mesophilic organisms", which in the present context is organisms having optimum growth temperatures at about 30°C and "thermophilic organisms", which in the present context is organisms having optimum growth temperatures in the range of about 40 to about 45°C.

The selection of strains for the starter culture of the invention will depend on the particular type of fermented food or feed product to be manufactured. E. g. for cheese and butter manufacturing, mesophilic cultures of *Lactococcus* species, *Leuconostoc* species and *Lactobacillus* species are widely used. Thus in one embodiment the culture according to the invention comprises one or more mesophilic organisms having optimum growth temperatures at about 30°C. Typical organisms belonging to such mesophilic organisms include *Lactococcus lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis, Lactobacillus casei* subsp. casei and *Lactobacillus paracasei* subsp. *paracasei.*

In yet another embodiment of the invention the culture according to the invention comprises one or more thermophilic organism(s) having optimum growth temperatures at about 40°C to about 45°C. Thermophilic organisms are frequently used to produce yoghurt and other fermented milk products, but also certain cheese are produced by use of thermophilic cultures,e.g. emmentaler cheese and special Italian cheeses. Typical organisms belonging to such Thermophilic organisms include organisms selected from the group comprising *Streptococcus thermophilus, Enterococcus faecium, Lactobacillus delbrueckii* subsp. *lactis, Lactobacillus helveticus, Lactobacillus delbrueckii* subsp. *bulgaricus* and *Lactobacillus acidophilus.*

In particular, lactic acid bacteria cultures (LAB-cultures) have found widely commercial use. Thus a preferred embodiment of the invention is a LAB-culture that comprises one or more organisms selected from the group comprising *Lactococcus* spp., *Streptococcus* spp., *Enterococcus* spp., *Lactobacillus* spp., *Leuconostoc* spp., *Pediococcus* spp. and *Bifidobacterium* spp.

Commercial starter cultures are frequently categorized according to their applications. An *O-culture* is used to make cheese without holes (Cheddar, Cheshire, Feta) and typically comprises one or more organisms selected from the group comprising *Lactococcus lactis* subsp. *lactis* and *Lactococcus lactis* subsp. *cremoris.* A *D-culture* is used to make butter and typically comprise one or more *Lactococcus* species i.e. *Lactococcus lactis* subsp. *lactis, Lactococcus lactis* subsp. *cremoris* and *Lactococcus lactis* subsp. *lactis* biovar. *diacetylactis.. A L-culture* can conveniently be used to produce cheese with only small holes (cottage cheese) and curdled milk products with low $CO_2$-production. Typically organisms in an L-culture are *Lactococcus lactis* subsp. *lactis, Lactococcus lactis* subsp. *cremoris* and *Leuconostoc mesenteroides* subsp. *cremoris.* And finally a *LD-culture* is used to make cheese with normal hole sizes, curdled milk products (junket) and sour butter. Commercially, a LD-culture is currently one of the most used mixed *cultures.* A *LD-culture* typically comprises one or more organisms selected from the group comprising *Lactococcus lactis* subsp. *lactis, Lactococcus lactis* subsp. *cremoris, Lactococcus lactis* subsp. *lactis* biovar. *diacetylactis* and *Leuconostoc mesenteroides* subsp. *cremoris.*

As is the case of other types of mixed starter cultures the specific amount of the individual bacterial species in a *LD-culture* may vary in accordance with the specific required use. The skilled person is aware of this and capable of determining the preferred mixed culture composition according to the required needs.

For instance, if aroma is required an optimal composition of the aroma making bacteria *Lactococcus lactis* subsp. *lactis* biovar. *diacetylactis* and *Leuconostoc mesenteroides* subsp. *cremoris* should be preferred.

A preferred LD-culture comprises:

Table 1.

| LD-culture composition. | |
|---|---|
| *Lactococcus lactis* subsp. *lactis, Lactococcus lactis* subsp. *cremoris* | 60 - 95 %, preferably 70 - 90% |
| *Lactococcus lactis* subsp. *lactis* biovar. *diacetylactis, Leuconostoc mesenteroides* subsp. *cremoris* | 5 - 40 %, preferably 0,1 to 30 % |

Within the ranges above, it is preferred to have from 0.25 to 6% of *Leuconostoc mesenteroides* subsp. *cremoris* and from 7 to 30% of *Lactococcus lactis* subsp. *lactis* biovar. *diacetylactis.*

Of course the total percentage sum of the 4 different LAB specifies cannot exceed 100%. However, it may be less than 100% if other bacteria than the 4 mentioned ones are present in the *LD-culture.* Example 2 herein provides an example of a stabilized *LD-culture.*

Fungal cultures are another group of microbial starter cultures, which may be used in accordance with the invention. Fungal cultures, such as yeast cultures and cultures of filamentous fungi, are commonly used in the manufacture of certain types of cheese and beverage. Examples of currently used cultures of fungi include *Penicillium roqueforti*, *Penicillium candidum, Geotrichum candidum*, *Torula kefir*, *Saccharomyces kefir* and *Saccharomyces cerevisiae.*

A particular preferred embodiment of the present invention is culture comprising *Lactobacillus acidophilus.*

In a further aspect the invention provides a method for making a frozen culture comprising following steps: 1) adding the cryoprotective agent selected from the group consisting one or more compound(s) involved in the biosynthesis of nucleic acids or one or more derivative(s) of any such compounds to viable organisms, 2) freezing the material to get frozen material, and 3) packing the frozen material in a suitable way.

It will be understood that the freezing and packing steps can be performed in a multitude of ways.

The freezing step should be optimised to ensure the cell's survival. Certain cells (e.g. most LAB) may be directly frozen, that is, brought directly into contact with an agent already at cryopreservation temperature. Direct methods include dripping, spraying, injecting or pouring cells directly into a "cryogenic temperature" fluid such as liquid nitrogen, liquid $CO_2$, or liquid helium. In the present context cryogenic temperatures refer to temperatures below -50°C, preferentially to temperatures below -150°C (123°K). Cells may also be directly contacted to a chilled solid, such as a liquid nitrogen frozen steel block. The cryogenic fluid may also be poured directly onto a container of cells. The direct method also encompasses contact cells with gases, including air, at a cryogenic temperature. A cryogenic gas stream of nitrogen or helium, may be blown directly over or bubbled into a cell suspension. Indirect method involved placing the cells in a container and contacting the container with a solid, liquid, or gas at cryogenic temperature. The container for the indirect freezing method do not have to be impermeable to air or liquid. For example, a plastic bag or a Tetra-Pak ® are adequate.

In one preferred embodiment, the culture is concentrated, eg. by centrifugation or ultrafiltration, the cryoprotective agent(s) is(are) added to the culture and the culture is subsequently added drop wise into liquid $N_2$ forming frozen culture granula. The frozen culture granula is then collected and packed in order could to be provided to the user. The frozen culture granula may be packed in a bottle, a tetra-pack ®, a bag, or any container which is suitable for the purpose. The frozen and packed culture granula are typically kept and distributed at temperatures which ensures that they stay frozen until they are to be used for inoculation of the media to be fermented or processed.

In yet a further aspect the invention provides a method for making a freeze dried culture comprising following steps: 1) adding a cryoprotective agent selected from the group consisting one or more compound(s) involved in the biosynthesis of nucleic acids or one or more derivative(s) of any such compounds to viable organisms, 2) freezing the material to get frozen material, 3) sublimation of water from the frozen material, and 4) packing the freeze dried material in a suitable way. The addition of cryoprotective agent(s), the optional concentration step, the freezing (or freeze-drying) and the packing steps can be performed as described.

Whereas frozen cultures need to be shipped and stored at low temperatures freeze-dried or lyophilised cultures can be shipped and stored without refrigeration for extended periods of time, provided that they are kept at dry conditions. However even in the case of freeze-dried cultures, storage below 0°C is recommended.

Typically both frozen and freeze-dried cultures according to the invention are provided as commercial DVS® -starter or Redi-Set® cultures. One advantage of the DVS®-starter cultures is that they may be added directly to the medium containing production fermentor or container in the form of frozen or freeze-dried cells. This results in an almost instantaneous regeneration of viable cells. Many of the commercially distributed starter cultures are lactic acid bacteria cultures, thus, a preferred embodiment of the present invention is the frozen or freeze-dried lactic acid bacteria (LAB) culture obtained as described.

In a further aspect, the invention pertains to a method of preparing a food or a feed product said method comprising using a frozen of freeze-dried culture according to the invention.

In a specific embodiment the food product is a milk-based product such as cheese, yoghurt, butter or a liquid fermented milk product, such as e. g. buttermilk or drinking yoghurt. Furthermore, the food product may be selected from a meat product, a vegetable product and a beverage such as wine and beer.

Another significant application of the method according to the present invention is the use of the liquid starter cultures as so-called probiotics. By the term "probiotic" is in the present context understood a microbial culture which, when ingested in the form of viable cells by humans or animals, confers an improved health condition, e. g. by suppressing harmful micro-organisms in the gastrointestinal tract, by enhancing the immune system or by contributing to the digestion of nutrients. A typical example of such a probiotically active product is "sweet acidophilus milk".

In further embodiments, the method according to the invention is used in the production of an animal feed such as silage e. g. grass, cereal material, peas, alfalfa or sugar-beet leaf, where bacterial cultures are inoculated in the feed crop to be ensiled in order to obtain a preservation hereof, or in protein rich animal waste products such as slaughtering offal and fish offal, also with the aims of preserving this offal for animal feeding purposes.

The invention presented in the form of claims

Preferred aspects and embodiments of the invention may be presented in the form of so-called claims. This is given below.

1. A frozen or freeze-dried culture, characterized in that the culture comprises one or more cryoprotective agent (s) selected from the group consisting one or more compound(s) involved in the biosynthesis of nucleic acids or one or more derivative(s) of any such compounds.

2. The culture according to claim 1, wherein the one or more cryoprotective agent(s) is/are selected from the group of purine bases, pyrimidine bases, nucleosides and nucleotides.

3. The culture according to claim 2, wherein the one or more cryoprotective agent(s) is/are selected from the group of nucleotides.

4. The culture according to claim 3, wherein the one or more cryoprotective agent(s) is/are selected from the group of nucleoside monophosphates.

5. The culture according to claim 4, wherein the one or more cryoprotective agent(s) is/are selected from the group of IMP, GMP and AMP.

6. The culture according to claim 5, wherein the cryoprotective agent is inosine-5'-monophosphate (IMP).

7. The culture according to claims 1 to 6, wherein the cryoprotective agent is an agent or mixture of agents, which in addition to its cryoprotectivity has a booster effect.

8. The culture according to claims 1 to 7, wherein the culture comprises from about 0.1% to about 20% of the cryoprotective agent or mixture of agents measured as %w/w of the frozen material.

9. The culture according to claim 8, wherein the culture comprises from about 2% to about 5% of the cryoprotective agent or mixture of agents measured as %w/w of the frozen material.

10. The culture according to any of the preceding claims, which is freeze dried.

11. The culture according to claims 1 to 10, wherein the culture is a starter culture.

12. The culture according to any of the preceding claims, wherein the culture comprises one or more organisms selected from the group comprising *Bifidobacterium* spp., *Brevibacterium* spp., *Propionibacterium* spp., *Lactococcus* spp. including *Lactococcus lactis* subsp. *lactis* and *Lactococcus lactis* subsp. *cremoris, Lactobacillus* spp. including *Lactobacillus acidophilus, Streptococcus* spp., *Enterococcus* spp., *Pediococcus* spp., *Leuconostoc* spp., *Oenococcus* spp. and fungal spp. including *Pencillium* spp., *Cryptococcus* spp., *Debraryomyces* spp., *Klyveromyces* spp. and *Saccharomyces* spp.

13. The culture according to claims 11 or 12, wherein the starter culture is a lactic acid bacteria (LAB) culture.

14. The culture according to any of claims 11 to 13, wherein the culture comprises one or more mesophilic organisms having optimum growth temperatures at about 30°C.

15. The culture according to claim 14, wherein the culture comprises one or more mesophilic organisms selected from the group comprising *Lactococcus lactis, Lactococcus lactis* subsp. *cremoris, Leuconostoc mesenteroides* subsp. *cremoris, Pediococcus pentosaceus, Lactococcus lactis* subsp. *lactis* biovar. *diacetylactis, Lactobacillus casei* subsp. *casei* and *Lactobacillus paracasei* subsp. *paracasei.*

16. The culture according to claims any of claims 11 to 13, wherein the culture comprises one or more thermophilic organisms having optimum growth temperatures at about 40°C to about 45°C.

17. The culture according to claim 16, wherein the culture comprises one or more thermophilic organisms selected from the group comprising *Streptococcus thermophilus, Enterococcus faecium, Lactobacillus delbrueckii* subsp. *lactis, Lactobacillus helveticus, Lactobacillus delbrueckii* subsp. *bulgaricus* and *Lactobacillus acidophilus.*

18. The culture according to claims 13, wherein the culture is a LAB-culture that comprises one or more organisms selected from the group comprising *Lactococcus* spp., *Streptococcus* spp., *Enterococcus* spp., *Lactobacillus* spp., *Leuconostoc* spp., *Pediococcus* spp. and *Bifidobacterium* spp.

19. The culture according to any of claims 12 to 18, wherein the culture is a LD-culture that comprises one or more organisms selected from the group comprising *Lactococcus lactis* subsp. *lactis, Lactococcus lactis* subsp. *cremoris, Lactococcus lactis* subsp. *lactis* biovar. *diacetylactis* and *Leuconostoc mesenteroides* subsp. *cremoris.*

20. The culture according to claims 12 to 18, wherein the culture is an O-culture that comprises one or more organisms selected from the group comprising *Lactococcus lactis* subsp. *lactis* and *Lactococcus lactis* subsp. *cremoris.*

21. The culture according to any of the preceding claims, wherein the culture is a culture comprising *Lactobacillus acidophilus.*

22. A method for making a frozen culture comprising following steps:

    I. adding the cryoprotective agent to viable organisms of any of the preceding claims,
    II. freezing the material to get frozen material, and
    III. packing the frozen material in a suitable way.

23. A method for making a freeze dried culture comprising following steps:

    I. adding a cryoprotective agent to viable organisms according any of claims 1 to 21,
    II. freezing the material to get frozen material,
    III. sublimation of water from the frozen material, and
    IV. packing the freeze dried material in a suitable way.

24. The frozen or freeze-dried lactic acid bacteria (LAB) culture obtainable by the methods of claims 22 or 23.

25. A method of preparing a food and a feed product, said method comprising using a culture according to any of claims 1-24.

26. The method according to claim 25, wherein the food product is selected from a milk based product, a meat product, a vegetable product and a beverage.

The invention is further illustrated in the following non-limiting examples and the figures wherein

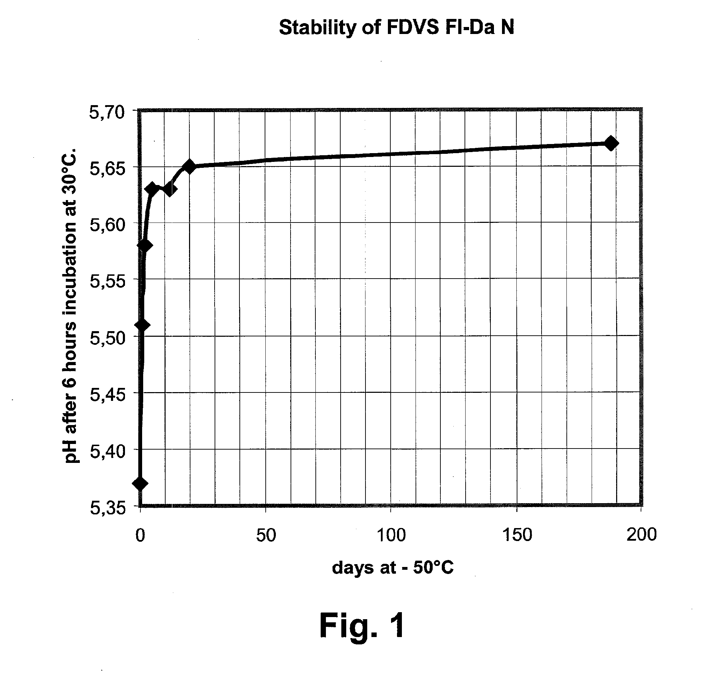

Fig 1. Shows the stability of a commercial frozen concentrated culture (F-DVS Fl-Da N, Chr. Hansen A/S Item. No. 501691) during the initial phase of storage, amount of inoculation material: 0.01% w/v. Note: a higher pH after 6 hrs. of incubation at 30°C is indicative of less acidifying activity (i.e. less metabolic activity) of the culture.

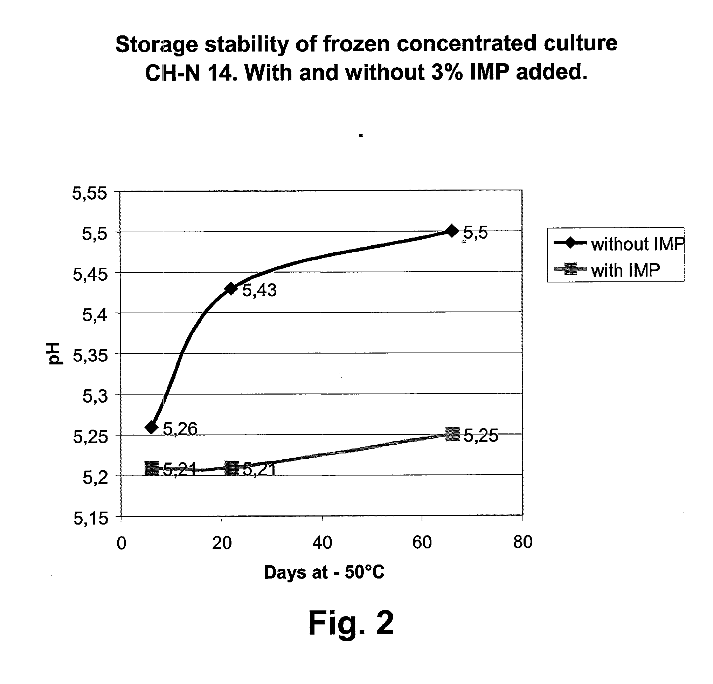

Fig 2. Shows the storage stability expressed as the acidifying activity of frozen concentrated culture F-DVS CH-N 14 (Chr. Hansen A/S Item. No. 200118). with and without 3% w/w IMP added. Note: the abscissa shows the pH measured after 6 hrs. of incubation at 30°C, amount of inoculation material: 0.01% w/v. A higher pH is indicative of less acidifying activity (i.e. less metabolic activity) of the culture.

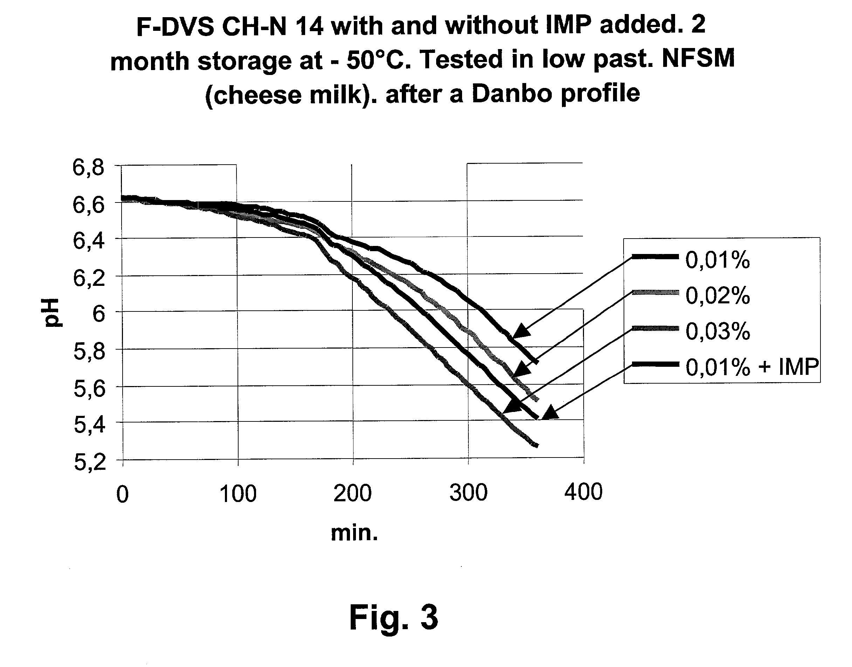

Fig 3. Shows the acidifying activity of F-DVS CH-N 14 cultures (Chr. Hansen A/S Item. No. 200118) with and without IMP added. The fermentation was performed with cultures after 2 months of storage at - 50°C. The fermentation was tested in low-pasteurized full milk following the Danbo temperature-profile of Table 2. The amounts of culture added is given in % (w/w). Note: the abscissa shows the pH measured after 6 hrs. of incubation at 30°C. A higher pH is indicative of less acidifying activity (i.e. less metabolic activity) of the culture.

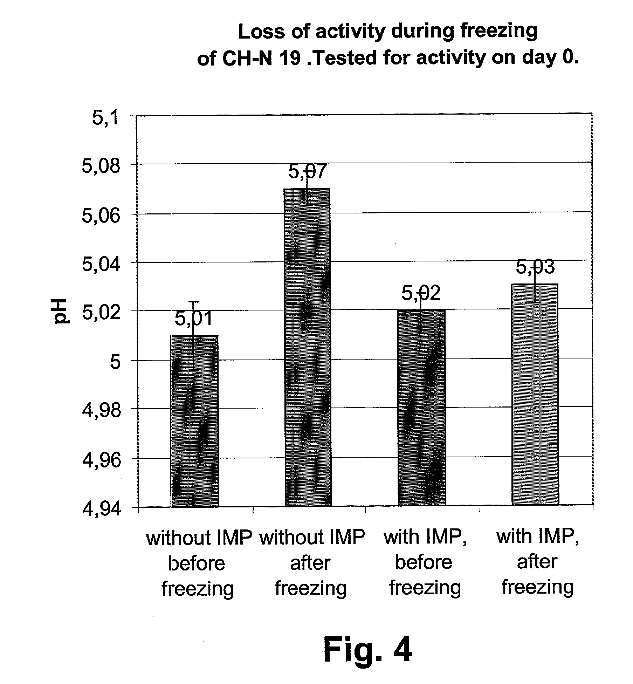

Fig 4. Illustrates loss of activity during freezing of the F-DVS CH-N 19 culture (Chr. Hansen A/S Item. No. 501593). The culture was tested for activity same as the culture was produced (on day 0). Error bars indicate the standard deviation. Note: the abscissa shows the pH measured after 6 hrs. of incubation at 30°C, amount of inoculation material: 0.01% w/v. A higher pH is indicative of less acidifying activity (i.e. less metabolic activity) of the culture.

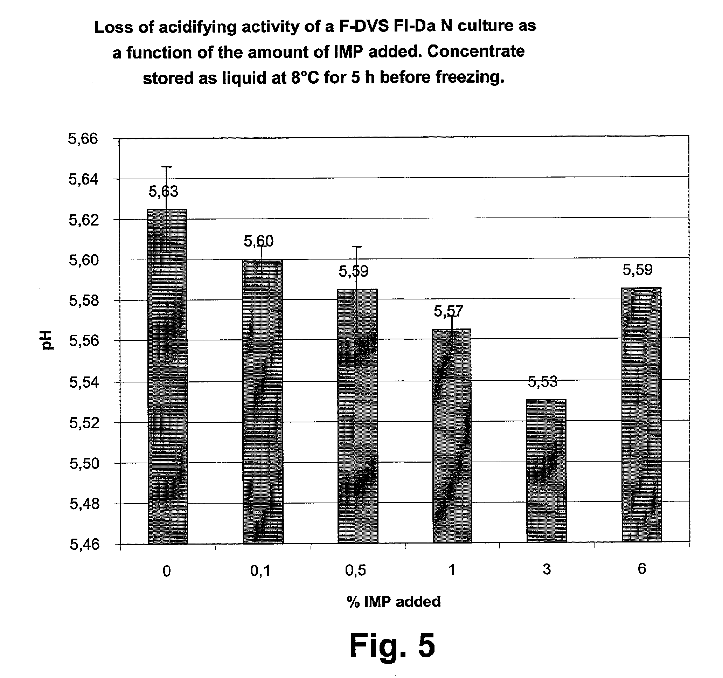

Fig 5. Illustrates the loss of acidifying activity of a F-DVS Fl-Da N culture (Chr. Hansen A/S Item. No. 501691) as a function of the amount of IMP added. Concentrates were stored as liquid at 8°C for 5 h before freezing. Error bars indicate the standard deviation. Note: the abscissa shows the pH measured after 6 hrs. of incubation at 30°C, amount of inoculation material: 0.01% w/v. A higher pH is indicative of less acidifying activity (i.e. less metabolic activity) of the culture.

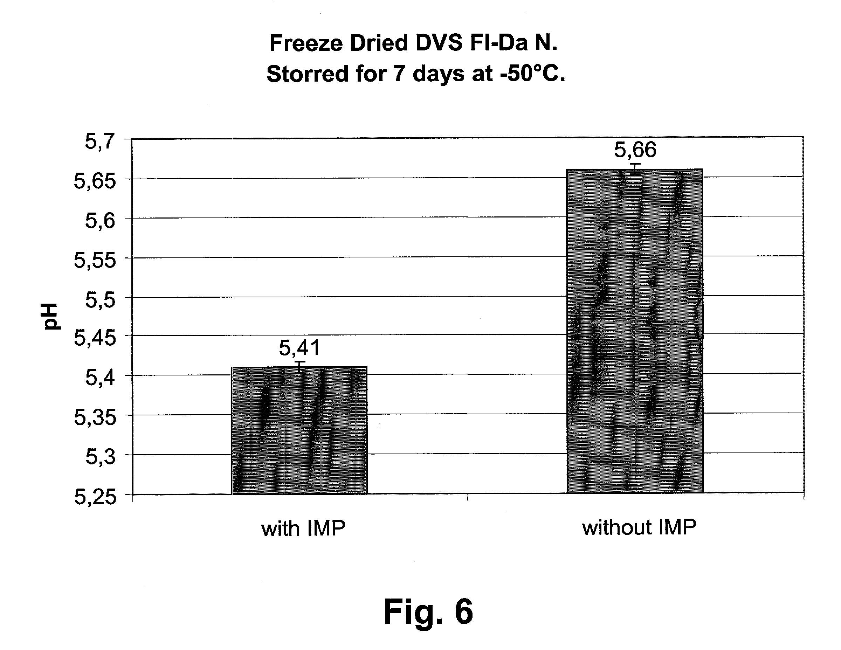

Fig 6. Shows the storage stability expressed as the acidifying activity of freeze-dried concentrated culture Fl-Da N culture with and without 3% w/w IMP added. Error bars indicate the standard deviation. Note: the abscissa shows the pH measured after 6 hrs. of incubation at 30°C, amount of inoculation material: 0.01% w/v. A higher pH is indicative of less acidifying activity (i.e. less metabolic activity) of the culture.

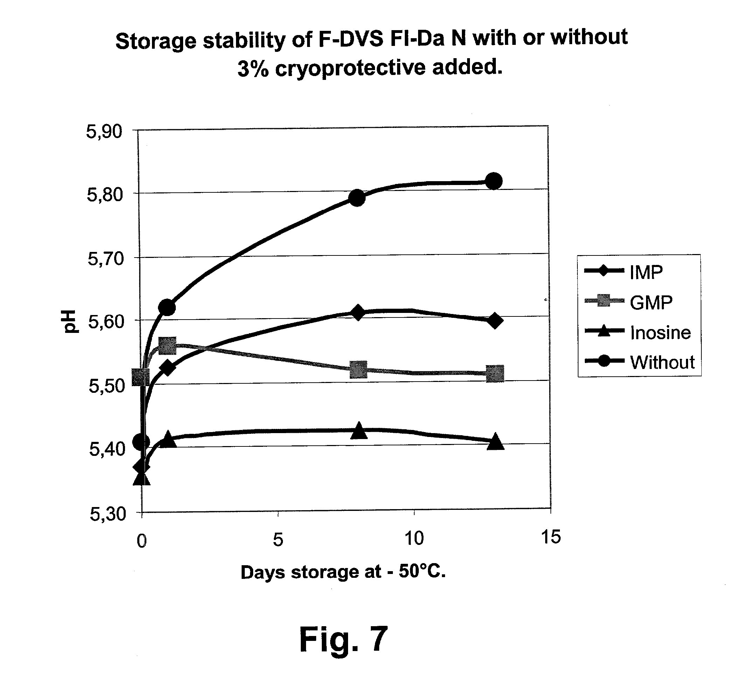

Fig 7. Shows the storage stability expressed as the acidifying activity of frozen concentrated culture (F-DVS Fl-Da N, Chr. Hansen A/S Item. No. 501691) during the initial phase of storage with or without 3% w/w IMP, GMP, Inosine or nothing added. Note: the abscissa shows the pH measured after 6 hrs. of incubation at 30°C, amount of inoculation material: 0.01% w/v. A higher pH is indicative of less acidifying activity (i.e. less metabolic activity) of the culture.

**EXAMPLES:**

**Materials and methods**

*Cultures:*

The following 3 cultures have used: Fl DaN, CH N 14 and CH N19. All three cultures are commercially available frozen LD-cultures in the form of Frozen Direct Vat Cultures (F-DVS), from Chr. Hansen A/S, Denmark as: F-DVS Fl-Da N (Chr. Hansen A/S Item. No. 501691), F-DVS CH-N 14 (Chr. Hansen A/S Item. No. 200118) and F-DVS CH-N 19 (Chr. Hansen A/S Item. No. 501593).

***Fermentation media and fermentation conditions:***

*Medium composition for culture of LD-cultures:*

The LD-cultures were cultured in a medium having the following composition: Casein hydrolysate (Oxoid, Basingstoke, UK, Product Code L41), 30 g/l; Primatone RL (Quest, Naarden, The Netherlands, Product Code 5X59051), 30 g/l; soya peptone (Oxoid, Basingstoke, UK, Product Code L44), 30 g/l; yeast extract (Oxoid, Basingstoke, UK, Product Code L21), 15 g/l; MgSO4, 1.5 g/l; Na-ascorbate, 3 g/l; and lactose 50 g/l.
The medium was sterilized by UHT-treatment (143°C for 8 sec.). The finished medium had a pH of 6.5.

*Fermentation condition for LD-cultures:*

The fermentation was performed in a 100 l fermentation tank at 30°C using 1 % (w/w) of the culture mentioned above as inoculum. The anaerobic fermentation was run with nitrogen in the headspace and a headspace pressure of about 0.2 bar. The cultures were allowed to acidify to pH 6.0. The pH was subsequently maintained at 6.0 by controlled addition of 13.4 N $NH_4OH$.
When no further base consumption was detected, the respective culture was cooled down to about 10°C.

*Postfermentation treatment of LD-cultures:*

Following cooling, the bacteria in fermentation broths were concentrated 10-20 times by centrifugation and subsequently frozen as pellets in liquid nitrogen at one atmosphaere of pressure. The acidifying activity of pellets were measured at various times after freezing the rest of the pellets were stored at - 50°C until further analysis.

***Acidifying activity assay and CFU analysis:***

Frozen culture was inoculated on a 0.01 % (w/w) level in 200 ml UHT-sterilized reconstituted skimmed milk (RSM) containing 9.5% (w/w) solid matter and heated at 99°C for 30 minutes (LAB-milk). The and RSM were incubated at 30°C for 6h to permit acidification of the substrate material. The acidification activity was measured as described in Example 6: Analytical Procedure QAm-052, "acidification activity - UHT", Chr. Hansen A/S (Denmark).

*Simulated cheese production after a DANBO temperature-profile:*

The acidification is performed according to a temperature profile reflecting the temperature time-course which the culture will typically encounter when used in the dairy for production of a given dairy product in this case the DANBO cheese.
pH is measured at a fixed times as indicated in table 2

### Table 2. The Danbo-profile

| Time, minutes | Temperature, °C | Variation |
|---|---|---|
| 02:40 | 31.0°C | ±0.2 °C |
| 00:15 | Ramp 31.0°C to 38.0°C | ±0.5 °C |
| 00:35 | 38.0°C | ±0.2 °C |
| 04:24 | Ramp 38.0°C to 16.0°C* | ±0.5 °C |
| up to 16:00 | 16.0°C | ±0.2 °C |

The acidification activity was measured as described in example 7: Analytical Procedure QAm-043, acidification activity - "Programmed temperature profile" Chr. Hansen A/S (Denmark).

CFU analysis was measured and calculated as described in example 8: Analytical Procedure Q-AM-071, "Enumeration of microorganisms" Chr-Hansen A/S (Denmark).

**Example 1: Stability study of frozen LD-culture of FI-Da N.**

In this example the stability measured by the acidification activity of a commercially produced LD-culture: F-DVS FI-Da N (Chr. Hansen A/S Item. No. 501691) was followed over a period of 6 months. The culture was produced and stored at -50°C as described in the Materials and Methods section.

In contrast to what is common the first activity-measurement in this example was performed immediately after the culture were frozen as pellets in liquid nitrogen and followed by measurements after 1, 2, 12, 20 and 188 days of storage at -50°C.

The results of this experiment is shown in figure 1.

The result was very surprising to the present inventors. It was surprising that the acidification activity was drastically decreased during the very first few days of storage. After only one week of storage the acidification activity was reduced with 0.26 pH unit. This reduction is equivalent to a 50% reduction of the acidification activity after only one week of storage. After two weeks of storage further loss of the cultures acidification activity became less pronounced and the acidification activity of the culture only decreased marginally during the rest of the period.

The unexpected result of this experiment made the inventors realize that there were significant and hitherto unrecognized stability problems which related to the freezing and the initial phase of the storage of some types of commercial relevant concentrated frozen lactic acid bacteria cultures, such as e.g. commercial available frozen LD-cultures.

**Example 2: Stability study of frozen LD-culture of CH N14 using IMP as cryoprotective agent.**

This example describes the stability study with frozen direct vat set cultures (F-DVS) of CH N14 formulated with IMP as cryoprotective agent. In the experiments the concentration of IMP were kept at 3% w/w per gram concentrated biomass. The IMP were added to the concentrate as a 30% w/w sterile solution.

After fermentation, biomass was harvested and concentrated via centrifugation from fermentation broth of F-DVS CH N 14. The cell concentrate was divided into two portions of 300 gram each and IMP was added to one of the portion. The additives and concentrates were mixed for 30 minutes, frozen in liquid nitrogen and subsequently stored at -50°C. The frozen culture had a content of viable bacteria of at least $10^{10}$ colony forming units (CFU) per g frozen material. Culture activity in milk (LAB-milk) was measured the same day as the cultures were frozen and the activity was followed periodically up to 65 days.

Stability profiles for F-DVS of CH N14 given as acidification activity are summarized in figure 2.

It is evident that F-DVS CH N14 free of additives are loosing activity thus stability. The reduction in stability is equal to 0.25 pH units for CH N 14 after storage for 65 days at - 50°C. 0.25 pH units is nearly equal to a 50% loss of acidification activity.

**Example 3: Stability study of frozen LD-culture of F-DVS CH N14 using IMP as cryoprotective agents tested after a temperature profile.**

In this experiment samples from the culture described in Example 2 were tested after storage for approximately two months at -50°C. The acidification activity was measured at several time points during the incubation according to a simulated "Danbo" temperature profile - see table 2. The fermentation medium was low pasteurized full milk similar to the milk that normally is used for commercial production of Danbo cheese.

The acidifying activity of cultures with and without inosine-5'-monophosphate (IMP) added prior to freezing was compared.

One set of bottles with low pasteurized full milk was inoculated with a frozen CH N14 culture without added IMP. In this case the amount of culture added was 0.01%, 0.02% and 0.03%, respectively (w/w%).

Another set of bottles with low pasteurized full milk was inoculated with a frozen CH N14 culture with 3% (w/w%) IMP added prior to freezing. In this case the amount of culture added was 0.01% (w/w%).

As seen in example 2 the culture without IMP lost approximately 50 % of the acidification activity which is equivalent to that a culture with IMP added has an activity that is nearly twice the activity compared to a similar amount of a similar culture without added IMP.

To illustrate the booster effect of IMP the sample without IMP added was inoculated using three different amounts of CH N14 culture. Judged from the results obtained in example 1 (i.e. that a little less than 50% of the activity is lost during storage of a culture without IMP added) one would expect that the acidification activity of a 0.01% inoculum of a culture with IMP added would be somewhere between the acidification activity of a 0.01% and a 0.02% inoculum of a culture without IMP added.

However, as illustrated in figure 3, this is not the case. The acidification activity of a 0.01% inoculum of a culture with IMP added turned out to be somewhere between the acidification activity of a 0.02% and a 0.03% inoculum of a culture without IMP added. This extra activity we ascribe to the booster effect of the added IMP.

This experiment show that addition of IMP to a culture results in a 2 - 2.5 X higher activity compared with the addition of a similar amount of a similar culture without IMP added.

The booster effect was not apparent in example 2, because in example 2 the milk had been subjected to a rather harsh heat sterilization procedure, i.e. LAB milk. In our experience, the booster effect is most pronounced in milk used for cheese fabrication since such milk typically is low-pasteurized milk.

**Example 4:Loss of activity during freezing of CH-N 19.**

This example describes the loss of activity during the freezing of a CH N19 culture formulated with IMP as cryoprotective agent. In the experiments the concentration of IMP were kept at 3% w/w per gram concentrated biomass (added as a sterile 30% w/w solution).

After fermentation, biomass was harvested and concentrated via centrifugation from the fermentation broth of the CH-N 19 culture. The cell concentrate was divided into two portions of 300 gram and IMP was added to one of the portions. The additives and concentrates were mixed for 30 minutes and subsequently 150 g of the two portions was frozen in liquid nitrogen. The culture had a content of viable bacteria of at least $10^{10}$ colony forming units (CFU) per g frozen material. Frozen and non-frozen cultures with and without added IMP were tested for their acidifying activities immediately after they were produced. Culture activity in milk (heat sterilized LAB-milk) was measured.

The results shown in figure 4 show an loss of acidifying activity of 0.06 pH unit if the culture was frozen without added IMP. A loss of 0.06 pH unit is equivalent to a 5 - 10% loss of acidifying activity. However if IMP was added to this culture no significant loss of activity was observed. The difference of 0.01 pH unit is of same size as the standard error as indicated by the error bars on the figure.

It is concluded that IMP also is able to act as an cryoprotective agent and increase the stability also of this type of culture during freezing.

**Example 5:Dose response for IMP using culture of F-DVS Fl-Da N**

This example describes the dose response study with frozen cultures (F-DVS) of Fl-Da-N formulated with IMP as cryoprotective agent. In the experiments the concentration of IMP were 0%, 0.1%, 0.5%, 1%, 3% and 6% w/w per gram concentrated biomass. The additive were added to the concentrate as a 30% sterile solution.

After fermentation, biomass was harvested and concentrated via centrifugation from fermentation broth of Fl-Da N. The cell concentrate was divided into 6 portions of 300 gram and IMP was added to each one of the portions. To simulate a situation similar to the industrial situation during the freezing process the additives and concentrates were mixed and stored for 5 hours at 8°C and subsequently frozen in liquid nitrogen and further stored at -50°C. Thus this example can not be compared with the previous examples. The frozen culture had a content of viable bacteria of at least $10^{10}$ colony forming units (CFU) per g frozen material. Culture activity in milk (LAB-milk) was measured the same day as the frozen cultures were formulated.

Results are shown in figure 5.

From these results it is clear that the concentrated cultures which were frozen without added IMP showed the largest loss of acidifying activity. The optimal result (i.e. smallest decrease of acidifying activity) was obtained with addition of 3% (w/w%) IMP.

**Example 6: Analytical Procedure QAm-052, "acidification activity - UHT", Chr. Hansen A/S (Denmark).**

APPLICATION

This method is used for determination of acidification activity.

PRINCIPLE

For F-DVS and FD-DVS products:

The culture is diluted and inoculated into milk. Incubate over a given time at a given temperature. After incubation pH is measured

For Frozen Redi-Set® (F-RS) and Freeze-dried Redi-Set® FD-RS products:

For these products, the activity analysis consists of 2 growth steps. A bulk starter is prepared by growing the culture in milk over a given time and temperature. After this the bulkstarter is inoculated in milk, and after a new incubation pH is measured.

ANALYSING PARAMETERS

Statement of the products analysing parameters can be read in Laboratory Information Management System (LIMS). Examples: Type of milk, Temperature of milk at 1st and 2nd weighing, Incubation time, Incubation temperature, Inoculation percent for the samples and control standards.

APPARATUS AND REAGENTS

pH-meter; pH electrode; Calibration buffers, pH $7.01 \pm 0.01$ and pH $4.00 \pm 0.01$; Water bath with a thermostate, precision $\pm 0.2°C$; Temperature sensor; Balance, precision 0.01 g with minimum two decimals; Rotation apparatus; Thermometer; Watch; Magnetic stirrer; Magnets; Beakers, 50 ml.

PROCEDURE

Preparation of analysis:

Note: All flasks should originate from the same batch i.e. with the same date.
At least 16 hours before start of analysis the lids on all bottles are loosened. Water bath/s is/are tempered to incubation temperature. Bottles for 1. weighing are tempered to inoculation temperature (can be either cold or hot milk). Buffer pH 4.01 and pH 7.00 are placed in water bath at incubation temperature at least 30 min before calibration of pH meter.
Note: For samples, which are placed in ice bath at 4°C before incubation, the heating of the water bath is started by a timer.

Preparation of samples before analysis

Frozen cultures: Frozen samples/control standards are before 1. weighing placed in a foam box with dry ice and are kept here till all weighings are done.

Frozen cultures, which are thawn before use:

For frozen products, where a whole carton is used, the product is thawn according to, see local instruction. After thawing the sample may be kept at 4°C for max. 30 min, before use. For frozen cultures in cans, a can is placed in a water bath at 22°C for 20 min in order to thaw the contents. After thawing the cultur may be kept at 4°C for max. 30 min. before use.

Freeze dried cultures:

Freeze dried samples/control standards are acclimatized at room temperature for at least 15 min before start of analysis.

Inoculation procedure

1st weighing/dilution:

The bottle for the 1st weighing is placed on the balance, which is set to zero.
Weighing of product/control standards is carried out directly into the milk
.Time for 1st weighing is always entered when inoculation is carried out in warm milk.The actual amount of inoculum (1st weighing) is entered with at least two decimals.
Frozen and thawn products are shaken carefully until the product has been distributed or max. 10 times, after which the bottle stands for approx 50 sec.
For freeze dried products the rotation apparatus (speed 2) is used for 5 min or until the products has been distributed.
Note: For freeze dried products of L. acidophilus, Bifidobacterium or L. casei, all inoculations are carried out in a clean bench.

2nd weighing

The bottle for the 2nd weighing is placed on the balance, which is set to zero.
For frozen and thawn produts, the dilution bottle is shaken carefully before 2nd weighing is carried out. The 2. weighing is carried out according to current quality control (Qc) procedures at time 1 minute.
For freeze dried products the 2nd weighing is carried out according to current Qc.
Time for 2nd weighing is entered when the inoculation are cold/warm.
The actual amount of inoculum (2nd weighing) is entered with at least 2 decimals.
The activity bottle is turned and the inoculation procedure is repeated for next samples/control.
Activity bottles which are inoculated from the same 1st weighing are inoculated in succession.
Note:Weighing off mixed products:
At first one 1 st weighing is prepared from each control standard/single strain. From each of these the 2nd weighings are carried out to the same activity bottle, so this will contain all control standards/single strains.
For frozen products the time from the first 1. weighing to the last 2. weighing must be max. 5 min. For freeze dried products the time from the first 1. weighing to the last 2. weighing must be max. 10 min.
At last place an uninolulated bottle of milk in the waterbath.
For products where 1st weighing takes place in cold milk:

$$\text{Tid}_{(measurement)} = \text{Tid}_{2.\ weighing} + \text{Tid}_{incubation}$$

or products where 1st weighing takes place in hot milk:

$$\text{Tid}_{(measurement)} = \text{Tid}_{1.\ weighing} + \text{Tid}_{incubation}$$

Note: For products which in addition are analysed for a long time acidification, the inoculation for this, can be carried out at the same time as the inoculation for acidification activity.
From 1st weighing used for acidification activity, 2nd weighing can be done in cold activity milk, which is placed at 4°C until incubation in a water bath which is heated to incubation temperature. In this case the bottles are incubated for ½ hour more than the given incubation time.
Note: For products where both inoculation and pH measurement of samples / Control standards, due to a long acidification time, is impossible within normal working hours, the 1st and 2nd weighing can be carried out in cold milk.
After inoculation of the activity milk, the bottles are placed in a water bath with cooling. The temperature sensor from a contact watch is placed in a bottle with uninoculated milk, which is placed in the water bath. A contact watch is connected to start heating of the water at a given time, and first when the temperature for the product of concern the incubation time starts.
Note: If it is necessary to use more than one water bath the Control standard MUST be incubated together with its connected samples in the same waterbath.

Measurement of pH electrode

Calibration is carried out according to current instructions regarding electrode calibration and maintenance.

Measurement of pH meter

pH must be measured in the samples/Control standards at Time $_{measurement}$. If time exceeded more than one minute, it is notes. If time is exeeded more than two minutes, the measuring is skipped.Just before time of measurement the bottle is turned 180°.

The pH measurement is carried out in the bottle or in a sample which is poured in a 50 ml beaker with magnet stirring.

pH is entered with at least 2 decimals.

Possible remarks on the measurement are entered. The measuring procedure is continued till all samples/control standards and the uninoculated milk are measured.The temperature of the water bath is measured in an uniculated bottle of milk and entered in the log book. Finally pH in calibrationbuffers are measured.

**Example 7: Analytical Procedure QAm-043, acidification activity - "Programmed temperature profile" Chr. Hansen A/S (Denmark).**

APPLICATION

This method is used for determination of acidification activity according to Pearce test and in other situations where acidification is performed according to a temperature profile e.g. Danbo-profile. Only Pearce test is included by the IDF standard.*international dairy standard*)

PRINCIPLE

The acidification is performed according to a temperature profile reflecting the temperature course, which the culture will typically encounter when used in the dairy for production of a given dairy product.

For Pearce test this is the cheese making temperature during the production of Cheddar.

pH is measured at a fixed time.

For cultures where rennet is not added during analysis, a continuous pH measurement may be applied.

ANALYSING PARAMTERS

Analysing parameters which are product specific are given in LIMS.

Definition of temperature profile (for products where Pearce profile is not used).

Control standard to be used.

Type of pH measurement.

Inoculation percents for sample and control standards.

Dilution milk: 206.9 g cold (4°C) LAB-milk (i.e. UHT-sterilized reconstituted skimmed milk (RSM) containing 9.5% (w/w) solid matter and heated at 99°C for 30 minutes).

Activity milk: 200g cold (4°C) low pasteurised whole milk 3.5% fat.

Rennet: Naturen® standard 190 diluted 1:40 with water.

APPARATUS AND REAGENTS

pH meter / pH meter for semi continuously pH measurement eks. Radiometer@ PHM92.

pH electrode Radiometer® PFC2401.

Buffers: pH 7.00 ± 0.01 and pH 4.01 ±0.01.

Water bath with a thermostat programmed for heating according to a predetermined temperature profile ± 0.2 °C.

Temperature sensor.

Balance, precision 0.01 g with minimum two decimals

Watch.

Magnetic stirrer.

Magnets

Beakers, 50 ml.

Small plastic cups.

Rotation apparatur.

PROCEDURE

Preparation of analyze
All bottles should be from the same batch i.e. with the same date.
Waterbath/s is/are tempered to the initial temperature of the temperature profile to be used.
Bottles for dilution (=1. weighing) and for activity (2. weighing) are placed at 4 °C until just before use.
Buffers pH 4.01 and pH 7.00 are placed in water bath at the specified measuring temperature ± 0.2 °C at least 30 min before calibration of pH meter.

Preparation of samples before analysis.

Frozen cultures:

Frozen samples/control standards are before 1. weighing placed in a foam box with dry ice and are kept here till all weighings are done.
Frozen cultures, which are thawn before use:
For frozen products, where a whole carton is used, the product is thawn according to current instructions.
After thawing the sample may be kept at 4°C for max. 30 min, before use.

Freeze dried cultures:

Freeze dried samples and control standards are acclimatized at room temperature for at least 15 min before start of analysis.
Provided that the sample are going to be used for retest the day after, it may be stored at +8°C.
Inoculation procedure
Weighing of product / control standard is carried out directly into the milk.
The actual amount of inoculum (1st weighing) is entered with at least two decimals.
Frozen and thawn products are turned carefully about 4 times, after which the bottle stands for approx. 50 sec.
For freeze dried products the rotation apparatus must be used. It has to be driven with frequent speed for 5 minutes or till the product is completely soluted. This is controlled by leaving the bottle on the table for a moment and then check the solution by looking in the bottom of the bottle.

Note:

If convenient for the working routine a cold, 1. weighing can stand at room temperature for max. 15 minutes before 2. weighing.

2nd weighing:

The dilution bottle is turned before 2. weighing is carried out.
The actual amount of inoculum (2nd weighing) is entered with at least 2 decimals.
The activity bottle is turned and the inoculation procedure is repeated for samples/control standards.
Activity bottles which are inoculated from the same 1st weighing are inoculated in succession.
2 ml rennet is added each bottle either before or after 2. weighing. After this the bottles are turned so the rennet been distributed.

Rennet is not added to Danbo-profile.

(Not IDF standard)

The bottles are subsequently incubated at one time, as described in 6.
In the end 2 uninoculated milk bottles are placed in a water bath. One for measuring of the water bath temperature and one for measuring pH in the blind milk.

INCUBATION

Note: When more water baths are required, the control standard with corresponding samples MUST be incubated in the same water bath.

All activity bottles are incubated at the same time in a pre-heated water bath at the defined starting temperature for the temperature profile.

The temperature profile is started at the same time as the bottles are placed in the water bath.

Hereafter the incubation temperature is controlled by a thermostat programmed for following a certain temperature profile. For Pearce test see table 3 and Danbo table 4.

The water level in the water bath should be min. 2 cm higher than the surface of the milk.

Table 3.

| Temperature programme in Pearce profile (following the IDF) | | |
|---|---|---|
| Time, minutes | Temperature, °C | Variation |
| 0 | 31.0 | ±0.2 °C |
| 50 | 31.0 | ±0.2 °C |
| 54 | 31.7 | ±0.5 °C |
| 58 | 32.2 | ±0.5 °C |
| 62 | 32.8 | ±0.5 °C |
| 66 | 33.3 | ±0.5 °C |
| 70 | 33.9 | ±0.5 °C |
| 73 | 34.4 | ±0.5 °C |
| 76 | 35.0 | ±0.5 °C |
| 79 | 35.6 | ±0.5 °C |
| 82 | 36.1 | ±0.5 °C |
| 85 | 36.7 | ±0.5 °C |
| 87.5 | 37.2 | ±0.5 °C |
| 90 | 37.8 | ±0.2 °C |
| 360 | 37.8 | ±0.2 °C |

Table 4:

| The Danbo-profile | | |
|---|---|---|
| Time, minutes | Temperature, °C | Variation |
| 02:40 | 31.0°C | ±0.2 °C |
| 00:15 | Ramp 31.0°C to 38.0°C | ±0.5 °C |
| 00:35 | 38.0°C | ±0.2 °C |
| 04:24 | Ramp 38.0°C to 16.0°C* | ±0.5 °C |
| up to 16:00 | 16.0°C | ±0.2 °C |

NOTE: On time 3 hours and 30 minutes, turn on the cooling water * Manually pH measurement after 06:00 hours +/- 2 minutes correspond to a temperature in the water bath of 25.5°C +/- 0.5°C.

CALIBREATION OF pH ELECTRODE

Calibration is carried out at initial temperature according to current instructions regarding electrode calibration and maintenance.

MEASUREMENT OF pH

After incubation the bottles are shaken well and pH is measured.

The pH measurement is carried out in the bottle or in a sample which is poured into a 50 ml beaker with magnet stirring.
pH is entered with at least 2 decimals.
Possible remarks on the measurement are entered.
The measuring procedure is continued till all samples / control standards and the uninoculated milk are measured.
Finally pH in buffers are measured and entered.

Continuous pH measurement

The pH values are sampled from the moment, the temperature profile is started. After the incubation is completed, the measured pH values in both buffers at initial temperature are registered.

**Example 8: Analytical Procedure Q-AM-071, "Enumeration of microorganisms" Chr-Hansen A/S (Denmark).**

AREA OF APPLICATION

This method is used for enumeration of lactic acid bacteria in various starter cultures and for counting of cross contaminants. The method is applicable only together with the concerned culture's analytical programme according to current quality control (Qc) procedures, why reference must be given to the analytical parameters herein.

PRINCIPLE

The method is a quantitative method where the result is reported as CFU/g.
A known amount of sample is homogenized with diluent and decimal dilutions are prepared. Appropriate dilutions are mixed with Leesment Agar or spread on the surface. After incubation all colonies are counted.

SAMPLING

Take samples according to established microbiological principles so that the sample is as representative as possible of the product to be examined.

APPARATUS AND GLASSWARE

Bottles á 250 ml
Tubes á 20 ml with caps
Autoclave, operating at $\pm$ 1°C
pH-meter sensitive to $\pm$ 0.2
Balance, operating at $\pm$ 0.01 g
Whirlmixer
Stomacher
Sterile Stomacher bags, 400 ml
Incubator, operating $\pm$ 1°C
Water bath, operating $\pm$ 1°C
Sterile pipettes
Petri dishes, 9 cm
Sterile Drigalski spatulas

MEDIA

Table 5.

| Diluent, Contents | |
|---|---|
| Casein peptone | 15.0 g |

Table 5. (continued)

| Diluent, Contents | |
| --- | --- |
| NaCl | 9.0 g |
| Antifoam FG-10. 2% | 1.14 ml |

Preparation

Suspend the ingredients in 1000 ml of distilled water. Heat to boiling point under frequent agitation. Dispense the diluent into bottles or tubes and autoclave at 121°C for 15 minutes.

pH after autoclaving: 7.0 ± 0.2.
Contents in bottles after autoclaving: 99.0 ± 1.0 ml.
Contents in tubes after autoclaving: 9.00 ± 0.05 ml.

If the diluent (Table 5) is to be used immediately then cool to 20°C or lower.

Storage

The prepared diluent (Table 5) may be stored for 6 months at 5°C in a dark place.

Table 6.

| Leesment Agar, Contents | |
| --- | --- |
| Tryptone (Oxoid L42) | 20.0 g |
| Yeast extract (Oxoid L21) | 5.0 g |
| Gelatine | 2.5 g |
| Lactose | 10.0 g |
| NaCl | 4.0 g |
| Tri-sodium-citrate, 2H2O (Merck 6432) | 2.0 g |
| Calcium lactate, 5H2O (Merck 2102) | 8.0 g |
| Agar (So-Bi-Gel) | 12.0 g |

Preparation

Suspend the ingredients in 1000 ml of distilled water. Heat to boiling point under frequent agitation till complete solution. Distribute the medium into bottles and autoclave at 121 °C for 15 min. pH after autoclaving: 6.8 ± 0.2.
If the medium is to be used immediately, cool it to approx. 47°C in a water bath. Before use 2 ml 50% glucose solution has to bee added to 200 ml of Leesment Agar (Table 6) for all CR-cultures.
Is the medium used for spread plating pour 12-15 ml of melted medium into Petri dishes and let the medium set and dry for 30 min in a Clean Bench.

Glucose solution

2.0 g glucose is thawed in 100 ml. distilled water. The solution is then sterile filtered by use of a 0.20 nM filter.

Leesment-Glucose Agar

Immediately before use 2 ml. of 50% glucose solution is added to a 200 ml. Leesment Agar (Table 6).

Storage

The prepared Leesment Agar (Table 6) may be stored dark for 6 months at 5°C. Poured plates packed in plastic bags may be stored dark for 10 days at 5°C.

PROCEDURE

NB - The analytical period from weighing out the sample until the sample is pour plated or spread plated must not exceed 30 min.

Before beginning the microbiological examination, melt the medium in a boiling water bath or by boiling in an autoclave, and then cool it to $47 \pm 1$ °C in a water bath.

Note - if prepoured plates are to be used the surface of the medium must be dry before plating.

In the Analytical Programme or Qc of the concerned product the following are given:

a) The amount of grammes (X) to be used for the first dilution (D1)
b) Minutes in Stomacher (M)
c) The appropriate dilutions (D2)
d) Amount to be seeded (A ml)
e) The incubation parameters
f) Plating method

Preparation of dilutions

Weigh X grammes of product into a sterile Stomacher bag and add by weighing the sufficient amount of sterile diluent to make the first dilution (D1). Place the bag in the Stomacher and treat for (M) minutes. If convenient, pour the contents of the bag into an empty, sterile bottle. By use of a sterile pipette transfer 0.1 or 1.0 ml from the lowest dilution into a bottle or tube with sterile diluent to make the next dilution (which now is the lowest!).

The contents in the bottle are mixed by shaking the bottle for 7 sec 20-25 times in an angle of 30°. The contents in the tube are mixed on a whirlmixer at maximum speed for 3x1 sec. Allow the foam to settle and repeat point 4 and 5 until the appropriate dilution/s (D2) is/are reached.

Pour plate

By use of a sterile pipette transfer A ml of the appropriate dilution/s (D2) into Petri dishes. Pour 10-12 ml of melted medium at not more than $47 \pm 1$°C, into each Petri dish and mix well with the sample. Pour 10-12 ml of melted medium into an empty Petri dish as a control of sterility. Leave the dishes on a clean horizontal surface until the medium has set. Invert the dishes and incubate according to the concerned products Qc.

Spread plate

By use of a sterile pipette transfer A ml of the appropriate dilution/s (D2) on to the surface of the medium. Spread the sample all over the medium by use of a sterile Drigalski spatula. Use an uninoculated Petri dish with medium as a control of sterility. Let the sample be absorbed by the medium before the dishes are inverted and incubated according to the concerned products Qc.

COUNTING OF COLONIES

For total viable cell counts Petri dishes containing between 30-300 colonies are chosen. All colonies are counted.

For counting of cross contaminants Petri dishes not containing more than 300 colonies are chosen. All colonies are counted. Note - By counting of cross contaminants the product to be analysed may produce pin point colonies which will make a cloud in the back ground. Therefore only colonies bigger than the pin point colonies in the cloud are counted.

CALCULATION

After counting a $\chi^2$ -test must be carried out on the plate counts according to standard statistical procedures.

Note - The $\chi^2$ -test is not carried out when the method is used for cross contaminants.

If the $\chi^2$ -test is not accepted the results must be rejected and the analysis repeated.
If the $\chi^2$ -test is accepted the mean number (N) of CFU/g is calculated according to below:

$$N = (\textstyle\sum c )/((n1+0.1n2)d)$$

where:

$\sum c$ is the sum of colonies counted on all Petri dishes;
n1 is the number of Petri dishes in the first dilution;
n2 is the number of Petri dishes in the second dilution;
d is the dilution factor corresponding to the first dilution.

**REPORTING OF RESULTS**

The calculated count may be reported as in the example above or as a rounded number with two significant digits.
Results which are reported externally should always be rounded.

Example

19 184 is rounded to 19 000 and is reported as $1.9 \times 10^4$.
$294 \times 10^8$ is rounded to $290 \times 10^8$ and is reported as $2.9 \times 10^{10}$.

For a three-digit number, round the third digit to the nearest zero: - If the third digit is 5 and the preceding digit is an even number, round the number down. If the preceding digit is an odd number round the number up.
Eksempel 28 500 is rounded to 28 000 and
11 500 rounded to 12 000

**Example 9: Stability study of a freeze-dried LD-culture of Fl-Da N.**

This example describes the stability of freeze-dried cultures (FD-DVS) of Fl-Da-N formulated with and without IMP as cryoprotective agent. In the experiments the concentration of IMP was 0% and 3% w/w per gram concentrated biomass. The additive were added to the concentrate as a 30% sterile solution.
After fermentation, biomass was harvested and concentrated via centrifugation from fermentation broth of Fl-Da N. The cell concentrate was divided into 2 portions of 300 gram and IMP was added to one of the portions. To simulate the situation encountered in the industrial situation during a freezing process, the additives and concentrates were mixed and stored for 5 hours at 8°C and subsequently frozen in liquid nitrogen and further stored at -50°C for one day before freeze-drying. After freeze-drying was completed the culture was stored at -50°C until analysis. The frozen culture had a content of viable bacteria of at least $10^{10}$ colony forming units (CFU) per g frozen material. Culture activity in milk (LAB-milk) was measured after 7 days of storage at -50°C.
Results are shown in figure 6.
It is evident that freeze-dried DVS Fl-Dn N without added IMP are loosing activity thus stability. The reduction in stability is equal to 0.25 pH units for Fl-Dn N after storage for 7 days at - 50°C. 0.25 pH units is nearly equal to a 50% loss of acidification activity.

**Example 10: Stability study of frozen LD-culture of F-DVS Fl-Da N using diferent compounds involved in the biosynthesis of nucleic acids as cryoprotective agents.**

This example describes the stability study with frozen direct vat set cultures F-DVS Fl-Da N (Chr. Hansen A/ S Item. No. 501691) formulated with either nucleotides IMP or GMP (guanosine-5'-monophosphate) or a nucleoside, Inosine as cryoprotective agent. In the experiments the concentration of IMP, GMP or Inosine were kept at 3% w/w per gram concentrated biomass. The IMP and GMP were added to the concentrate as a 25% w/w sterile aqueous solution, whereas the Inosine was added as dry powder. In the case of Inosine water was added to the culture in an amount which equals the amount added in the case of IMP or GMP addition.
After fermentation, biomass was harvested and concentrated via centrifugation from fermentation broth of F-DVS Fl-Da N. The cell concentrate was divided into four portions of 300 gram each and IMP, GMP, inosine or nothing was added to one of the portions. The additives and concentrates were mixed for 30 minutes, frozen in liquid nitrogen and subsequently stored at -50°C. The frozen culture had a content of viable bacteria of at least $10^{10}$ colony forming

units (CFU) per g frozen material. Culture activity in milk (LAB-milk) was measured the same day as the cultures were frozen and the activity was followed periodically up to 13 days.

Stability profiles for F-DVS Fl-Da N given as acidification activity are summarized in figure 7.

It is evident that F-DVS Fl-Da N free of additives are loosing activity thus stability. Relative to the culture stabilized by Inosine the reduction in stability of a culture without added Inosine is equal to 0.41 pH units for F-DVS Fl-Da N after storage for 13 days at - 50°C. 0.41 pH units corresponds to a 60% loss of acidification activity. Similarly the difference in stability between a F-DVS Fl-Da N culture with or without added GMP equals 0,31 pH units which corresponds to a 50% loss of acidification activity.

**REFERENCES:**

J K Andersen, B Fabech, B L Jacobsen, H Mejborn and L Rasmussen (1997) Biokontaminering, Veterinær- og fødevaredirektoratet, København, Denmark.

E W Nielsen and J A Ullum (1999). Mejerilære 1, Erhvervsskolernes Forlag, Odense, Denmark.

**Claims**

1. A frozen or freeze-dried culture, **characterized in that** the culture comprises one or more cryoprotective agent(s) selected from the group consisting one or more compound(s) involved in the biosynthesis of nucleic acids or one or more derivative(s) of any such compounds.

2. The culture according to claim 1, wherein the one or more cryoprotective agent(s) is/are selected from the group of purine bases, pyrimidine bases, nucleosides and nucleotides.

3. The culture according to claim 2, wherein the cryoprotective agent is inosine-5'-monophosphate (IMP).

4. The culture according to claims 1 to 3, wherein the cryoprotective agent is an agent or mixture of agents, which in addition to its cryoprotectivity has a booster effect.

5. The culture according to claims 1 to 4, wherein the culture comprises from about 0.1% to about 20% of the cryoprotective agent or mixture of agents measured as %w/wof the frozen material.

6. The culture according to any of the preceding claims, wherein the culture comprises one or more organisms selected from the group comprising *Bifidobacterium* spp., *Brevibacterium* spp., *Propionibacterium* spp., *Lactococcus* spp. including *Lactococcus lactis* subsp. *lactis* and *Lactococcus lactis* subsp. *cremoris, Lactobacillus* spp. including *Lactobacillus acidophilus, Streptococcus* spp., *Enterococcus* spp., *Pediococcus* spp., *Leuconostoc* spp., *Oenococcus* spp. and fungal spp. including *Pencillium* spp., *Cryptococcus* spp., *Debraryomyces* spp., *Klyveromyces* spp. and *Saccharomyces* spp.

7. The culture according to claim 6, wherein the culture comprises one or more mesophilic organisms having optimum growth temperatures at about 30°C.

8. The culture according to claims any of claims 1 to 6, wherein the culture comprises one or more thermophilic organisms having optimum growth temperatures at about 40°C to about 45°C.

9. A method for making a frozen culture comprising following steps:

   I. adding the cryoprotective agent to viable organisms of any of the preceding claims,
   II. freezing the material to get frozen material, and
   III. packing the frozen material in a suitable way.

10. A method for making a freeze dried culture comprising following steps:

   I. adding a cryoprotective agent to viable organisms according any of claims 1 to 8,
   II. freezing the material to get frozen material,
   III. sublimation of water from the frozen material, and
   IV. packing the freeze dried material in a suitable way.

Stability of FDVS FI-Da N

Fig. 1

**Storage stability of frozen concentrated culture
CH-N 14. With and without 3% IMP added.**

Fig. 2

F-DVS CH-N 14 with and without IMP added. 2 month storage at - 50°C. Tested in low past. NFSM (cheese milk). after a Danbo profile

Legend: 0,01% / 0,02% / 0,03% / 0,01% + IMP

Fig. 3

Loss of activity during freezing
of CH-N 19 .Tested for activity on day 0.

Fig. 4

EP 1 493 806 A1

Loss of acidifying activity of a F-DVS FI-Da N culture as a function of the amount of IMP added. Concentrate stored as liquid at 8°C for 5 h before freezing.

## Fig. 5

EP 1 493 806 A1

**Freeze Dried DVS Fl-Da N.**
**Storred for 7 days at -50°C.**

**Fig. 6**

# Storage stability of F-DVS FI-Da N with or without 3% cryoprotective added.

Fig. 7

**European Patent Office**

**EUROPEAN SEARCH REPORT**

Application Number

EP 03 07 7079

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 00 39281 A (HANSENS LAB ;KRAGELUND LENE (DK); KRINGELUM BOERGE (DK)) 6 July 2000 (2000-07-06) * page 6; claims 1-20 * | 1-10 | C12N1/04 C12N1/38 |
| X | WO 00 19817 A (CEDARS SINAI MEDICAL CENTER) 13 April 2000 (2000-04-13) * examples 2,3; table 1 * | 1,2,4,5, 9,10 | |
| X | DATABASE WPI Section Ch, Week 199401 Derwent Publications Ltd., London, GB; Class D15, AN 1994-002160 XP002262355 & JP 05 308956 A (MITSUBISHI HEAVY IND CO LTD), 22 November 1993 (1993-11-22) * abstract * | 1,2,4,5, 9,10 | |
| A | DASZYNSKI J ET AL: "STORAGE OF ERYTHROCYTES AT TEMPERATURES MINUS 20 TO MINUS 24 CELSIUS" ACTA MEDICA POLONA, vol. 22, no. 2, 1981, pages 151-160, XP009021344 ISSN: 0001-608X | | |

TECHNICAL FIELDS SEARCHED (Int.Cl.7)

C12N

The present search report has been drawn up for all claims

| Place of search | Date of completion of the search | Examiner |
|---|---|---|
| MUNICH | 21 November 2003 | Espen, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 03 07 7079

This annex lists the patent family members relating to the patent documents cited in the above-mentioned European search report.
The members are as contained in the European Patent Office EDP file on
The European Patent Office is in no way liable for these particulars which are merely given for the purpose of information.

21-11-2003

| Patent document cited in search report | | Publication date | Patent family member(s) | | Publication date |
|---|---|---|---|---|---|
| WO 0039281 | A | 06-07-2000 | AU | 1773700 A | 31-07-2000 |
| | | | WO | 0039281 A2 | 06-07-2000 |
| | | | EP | 1141233 A2 | 10-10-2001 |
| | | | PL | 349370 A1 | 15-07-2002 |
| WO 0019817 | A | 13-04-2000 | US | 6485959 B1 | 26-11-2002 |
| | | | EP | 1119244 A1 | 01-08-2001 |
| | | | WO | 0019817 A1 | 13-04-2000 |
| | | | US | 2003049840 A1 | 13-03-2003 |
| JP 5308956 | A | 22-11-1993 | JP | 3040507 B2 | 15-05-2000 |

EPO FORM P0459

For more details about this annex : see Official Journal of the European Patent Office, No. 12/82